(19) Europäisches Patentamt
European Patent Office
Office européen des brevets

(11) Publication number: 0 281 482 A1

# (12) EUROPEAN PATENT APPLICATION

(21) Application number: 88400505.9

(22) Date of filing: 03.03.88

(51) Int. Cl.[4]: **A 61 K 9/00**
A 61 K 9/52, A 61 K 9/70

(30) Priority: 06.03.87 US 22712

(43) Date of publication of application:
07.09.88 Bulletin 88/36

(84) Designated Contracting States:
AT BE CH DE ES FR GB GR IT LI LU NL SE

(71) Applicant: **RESEARCH TRIANGLE INSTITUTE**
**Post Office Box 12194 Research Tringle Park**
**Durham North Carolina (US)**

(72) Inventor: **Pitt, Colin G.**
**2801 Welcome Drive**
**Durham NC 27705 (US)**

(74) Representative: **Mongrédien, André et al**
**c/o SOCIETE DE PROTECTION DES INVENTIONS 25, rue de Ponthieu**
**F-75008 Paris (FR)**

## (54) Polymer blends for selective biodegradability.

(57) Biodegradation of a drug delivery system or temporary internal suture or device is controlled by preparing a blend of biodegradable polymers having distinct biodegradation rates. The resulting article has a unique biodegradation rate distinct from that of the component polymers. Predetermined or preselected biodegradation rates can be achieved by selection of appropriate blend components.

EP 0 281 482 A1

## Description

### Polymer Blends For Selective Biodegradability

#### Technical Field

This invention pertains to the area of technology concerned with biodegradable materials, particularly, materials suitable for use in a medical/surgical context, which are eroded or destroyed, over time, in the body. It is also directed to the controlled release of pharmaceutically active substances, such as medications and the like.

#### Background Art

Advances in medical technology have resulted in an increasing focus on the provision of biodegradable materials, suitable for use in vivo, particularly with an eye towards long term medical/surgical care. The application of these biodegradable materials, predominantly synthetic polymers and copolymers, has been varied. Among the most important applications are the preparation of biodegradable sutures and the like, which can be used to close wounds and incisions, or provide a temporary attachment for organs and implants, which are subsequently replaced by natural tissue growth. The sutures, being biodegradable, are slowly destroyed by the bodies natural enzymatic and fluid attack over time, removing the need for persistent, and sometimes invasive, medical supervision and treatment after the principle care functions have been completed. As a particular example, internal sutures, for closing incisions, and skin sutures, dissolve automatically, avoiding the need for a return visit as an outpatient, for removal of the sutures.

Another critically important application has been the use of these polymers as articles for achieving sustained release of pharmaceutical agents, particularly through implants. In this context, biodegradable polymers are loaded with at least one pharmaceutically active agent, which is slowly made available to the patient, through at least one of a plurality of mechanisms. Thus, the biodegradable material may be simply loaded with a pharmaceutical agent, and as the polymer is bio-absorbed or bio-destroyed, the agent is released. Alternatively, the biodegradable material may be porous, and contain the pharmaceutically active agent(s) in a reservoir, which is released over time by diffusion. The biodegradable implant is selected so as to degrade with or subsequent to the release of the drug.

In yet other applications, biodegradable materials are used to constitute, in whole or in part, temporary replacement structures, such as ligaments and the like, which are to be gradually destroyed and replaced with natural tissue. The structures may incorporate tissue growth augmentation materials, released as the material degrades.

Examples of all these embodiments abound in the art, and are not discussed in detail, herein. One feature that is common to all these applications, and is immediately apparent as a critical variable, is the biodegradability of these materials. Thus, to achieve a constant rate of release, or perhaps a desired rate of release for a certain pharmaceutical, or to ensure a sufficient life time for the implant to allow for replacement by natural tissue growth, the biodegradability of the implant must be carefully controlled, and selected to match the desired profile. It is, therefore, obviously desirable to be able to control the biodegradability of a biodegradable material to be used in an implant. Attempts have been made to control, or at least selectively predict, the biodegradability of various polymers suitable for in vivo applications of this type.

U.S. Patent 4,578,384 addresses a polylactic acid/polyglycolic acid copolymer which is biodegradable, utilized in the context of bone repair. The biodegradation rate of the polymer is altered by varying the component ratios of the copolymer. A similar approach is described in U.S. Patent 3,981,303, directed to a similar biodegradable system, for the controlled release of pharmaceuticals. Biodegradation is selectively altered in this application by the addition of a "disintegration agent", to accelerate bioerrosion. U.S. Patent 4,148,871 is directed to biodegradable compositions based on ε-caprolactones, which provide for sustained release of pharmaceuticals either from a matrix of the polymer, or a reservoir defined by the polymer. Again, the rate of biodegradation is a characteristic of dominating importance, and is selected or controlled by selective choice of the comonomers of the copolymer, changing the chemical nature of the resulting copolymer.

A totally different approach to control of biodegradation, and therefore, pharmaceutical release rate, is taken in U.S. Patents 4,180,646, 3,811,444 and 4,601,893. Each of these patents achieves alteration of the rate of release, and selective preparation of suitable implants having the desired release, by the provision of multiple layers of biodegradable polymers, each having a different copolymer composition, and therefore, a different biodegradation rate.

These systems, and others employed in the art, are not entirely satisfactory in providing biodegradable materials suitable for in vivo use which can be selectively prepared to achieve a desired biodegradation rate. In particular, the field of monomers, and therefore, copolymers, that are suitable for use inside the bodies of patients such as mammals, including humans, is severly limited due to toxicology data demonstrating a lack of available biocompatible materials. Thus, the range of copolymers that may be prepared is similarly limited. This limitation is further aggrevated by the fact that not all theoretically possible copolymers can be made, in actuality. Beyond relatively restrictive limitations, too much of one monomer or the other may result in a loss of physical characteristics, difficulty in handling, or simply, impossibility of preparation. Additionally,

copolymerization presents additional problems of solvent selection, processing and the like, which may not be consistent with the preparation of in vivo materials. Finally, copolymerization is, at best, an inexact art. European Patent Application 0058481 is directed to a method for continuous pharmaceutical release. While the bulk of the application is directed to the use of copolymers, there is a brief mention of the use of blends to achieve specific physical properties. There is nothing in the reference that suggests blending may alter biodegradation rate.

Accordingly, it remains an object of the art to prepare biodegradable materials having a preselected or desired biodegradation rate which can be predicted with reliability.

## Contemporaneous Developments

Recently, after development of this invention, the literature (Chang et al, Pharmaceutical Technology, October, 1986) has reported efforts to control permeability of drug-containing microcapsules by blending two or more polymers together (i.e., a lactone and a cellulosic nitrate). It should be noted that the only method of drug release contemplated or disclosed as effected by this process is permeation through the microcapsule wall. The article does not discuss any methods of controlling biodegradability.

Accordingly, a method of controlling, or predetermining, the biodegradation rate in a sustained release format or internal structure where biodegradation plays a dominant, or contributing role, remains a pressing need in the industry.

## Disclosure of the Invention

One object of this invention is to meet the above-identified needs of the industry, and overcome the obstacles encountered therein.

It is another object of this invention to provide a drug delivery system, wherein the rate of drug release is determined, at least in part, by biodegradation, which has a predetermined biodegradation rate achieved by blending of biodegradable polymers.

It is another object of this invention to provide a method whereby a drug delivery system having a controlled or predetermined biodegradation rate can be provided, involving the blending of biodegradable polymers.

It is the other further object of this invention to provide a drug delivery system comprised of a blend of biodegradable polymers, the biodegradability of the system being different from that of its component parts.

It is yet another object of the invention to provide biodegradable articles designed for use in vivo which are desirably degraded over time, such as sutures and the like.

These and other objects are achieved by preparing an intimate blend of two or more biodegradable polymers having distinct biodegradability values, as unblended polymers. Quite surprisingly, the biodegradation rate for the formed article is different from the rate of its component polymers, and in fact, is a rate for the article as a whole. Thus, while one of skill in the art would expect that a shaped article comprised of a blend of two or more polymers, subject to biodegradation, would, over time, eventually consist only of the slower degrading polymer, the quicker degrading polymer simply disappearing over time, in fact, an overall, distinct value is observed, and the component members of the polymer blend persist over time. This is particularly surprising because biodegradation is believed to be a function of chemical structure, which would not be effected by polymer blending.

## Best Mode For Carrying Out The Invention

Applicants have not determined the exact mechanism by which the biodegradability of the various polymers is altered when blended together into a shaped article for drug release, but the principle applied appears to be applicable to virtually any biodegradable polymer. Of particular interest are biodegradable polyesters, prepared from monomers such as lactones, dilactides, and lactic acid, as well as glycolic acid and glycolides in general. Many of the suitable acids have been designated, as a class within the class of polyesters, as poly-α-hydroxy acids. Commercially available forms of polylactic acid (PLA) and polyglycolic acid (PGA) are sold under the tradenames Dexon and Vicryl. The polymers used to prepare the blend of the claimed invention can be homopolymers, or copolymers of two or more biodegradable materials. The only requirement for use in the claimed invention is that the polymer be biodegradable, and have a biodegradation rate, as a pure polymer, distinct from that of the other members of the blends.

In general, biodegradation is the dominant form of release for sustained delivery of drugs of relatively high molecular weight, exceeding, e.g., about 500. The relatively high molecular weight of these drugs defeats or frustrates release through diffusion, controlled by polymer permeability. Additionally, where a temporary structure is contemplated such as a suture or scaffold, biodegradation is the only method controlling long term bioerosion.

To prepare the claimed invention, desirably, a blend of at least two biodegradable polymers is prepared. In general, the easiest method of preparation is to dissolve both in a common solvent. Alernatively, a melt blend may be prepared. From the blend, a wide variety of shaped articles can be provided. Included, without limitation, in the claimed invention are drug delivery systems comprising microcapsules, i.e., small capsules, 1-250 microns, comprised of an exterior wall of the polymer blend, containing drug on the inside; microspheres i.e., particles of the same size with a suspension or solution of the drug is distributed throughout the polymer; implants designed for sustained release over time, which may either define a sealed, interior reservoir where the drug is loaded, or may comprise a matrix of the polymer blend, with drug solubilized or

otherwise contained therein. While, in this embodiment, some diffusion of the drug from the matrix surface may be an important mechanism for drug release, under the above-noted constraints of molecular weight, biodegradation is likely to continue to be a contributing factor to the rate of drug release. In many applications, distinguishing between one form and another becomes a tautolgy. However, for ease of definition, microcapsules, microspheres and the like may be defined as having a capsule size less than 1 millimeter, where shaped articles having an interior reservoir greater than 1 millimeter in size are described, generically, as inserts.

Also contemplated are biodegradable articles for in vivo use, such as sutures, scaffolds and the like. These may or may not comprise absorbed pharmaceuticals, but are generally not characterized by interior reservoirs.

The blend of polymers may be comprised of virtually any ratio between the biodegradable polymers. However, in general, the greatest change in biodegradation rate, and therefore, the most marked effects of the claimed invention, are observed where, in a 2-component blend, both members are present in a range of 10%-90%. In a 3-member blend, a minimum presence of about 15% for each blend member is desirable. Although blends comprised of more than three polymers can be envisioned, it is unlikely that the desired biodegradation rate can only be achieved by such a complex mixture. Blending more than three polymers presents problems of compatibility, drug tolerance and the like.

Virtually any drug may be employed in the claimed invention. As noted, since the claimed invention concentrates on controlling the rate of biodegradation of the polymer containing the drug, the drugs, in general, will have a relatively high molecular weight, as drugs of lower molecular weight conventionally are released through diffusion (permeability). However, where the polymer employed is sufficiently glassy to frustrate diffusion of even lower molecular weight drugs, this invention may be advantageously employed. At molecular weights of about 500, many drugs are released through both diffusion through the polymer matrix, and the errosion of the polymer. Such an application falls within the invention, as claimed below.

Additionally, this invention provides a means for controlling the degradation of the polymer mass after the drug has diffused out entirely, or in siutations were drug release is entirely independent of biodegradation.

It can therefore be seen that the claimed invention, offers, for the first time, a method of controlling the biodegradation rate of drug delivery articles and systems and articles designed for short term survival in vivo which does not require complex adjustment of a variety of monomers in the preparation of a copolymer. Because there is no chemical reaction between the blend components of the claimed invention, the chief governing parameter is blend compatibility. The use of blends, as opposed to copolymers, also gives a far wider range of physical properties, particularly different from their corresponding copolymers.

## Example

Set forth below is an example of the invention, which is illustrative only, and not intended to limit the scope of the invention disclose herein.

A 1:1 blend of poly(ε-caprolactone) and poly(L-lactic acid-co-glycolic acid) was prepared by casting the polymers from a common solution in chloroform, and compression molding the resulting film. The rates of hyrolytic chain cleavage and weight loss of samples of this 1:1 blend were measured in phosphate buffered saline at 37°C under conditions that are known to simulate the in vivo biodegradation process. The results of this experiment, summarized in Table 1 and Figure 1, demonstrate that the rate of poly(ε-caprolactone) degradation is accelerated while the rate of degradation of poly(L-lactic acid-co-glycolic acid) is slowed when these polymers are blended. Since poly(L-lactic acid-co-glycolic acid) can be blended with poly(ε-caprolactone) in certain proportions without a marked change in permeability, relative to poly(ε-caprolactone), it is evident that such blending provides a means of selectively modifying the degradability with only minor changes in permeability.

Table 1. Rates of Hydrolytic Chain Cleavage of Poly(ε-caprolactone) and Poly(glycolic-co-L-lactic acid) as Pure Polymers and as a 1:1 Blend, as Reflected by Decrease of Molecular Weight ($M_n$) in Buffered Saline, pH 7.4

| | Time (Hours) | | | | | |
|---|---|---|---|---|---|---|
| Polymer | 0 | 235 | 500 | 739 | 1243 | 1752 |
| | | | Pure Polymer | | | |
| PCL | 83,200 | 79,250 | 76,200 | 79,800 | 71,100 | 73,800 |
| PGLA | 20,500 | 13,200 | 6,600 | 3,800 | 480 | - |
| | | | 1:1 Blend | | | |
| PCL | 51,400 | 45,600 | 41,000 | 32,500 | 28,600 | 24,000 |
| PGLA | 6,800 | 5,300 | 5,400 | 2,900 | 1,600 | 1,150 |

Obviously, numerous modifications and variations of the present invention are possible in light of the above teachings. It is therefore to be understood that within the scope of the appended claims, the invention may be practiced otherwise than as specifically described herein.

## Claims

1. An article for controlled drug delivery, wherein biodegradation of the article comprises at least one method whereby said article is used to achieve drug release, comprising:

a shaped article containing said drug and further comprised of a blend of at least two biodegradable polymers having distinct biodegradation rates, wherein the overall degradation rate of the shaped article is different from that of the individual polymers comprising the blend.

2. The article of Claim 1, wherein said blend is comprised of two biodegradable polymers, both present in a range of 10%-90%, by weight.

3. The article of Claim 1, wherein said drug has a molecular weight in excess of 500.

4. The article of Claim 1, wherein said polymers are comprised of homopolymers, copolymers or both.

5. The article of Claim 1, wherein said polymers are derived from monomers selected from the group

consisting of ε-caprolactone, DL- or L-lactic acid, and glycolic acid and are represented by one or more of the following chemical structures:

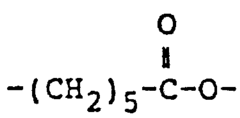

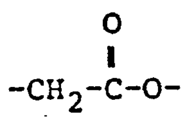

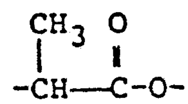

6. The article of Claim 1, wherein said article defines a microcapsule or microsphere, an implant in excess of 1/2 millimeter having a sealed internal reservoir, or polymer matrix with drug absorbed therein.

7. A shaped article for use as a temporary structure for in vivo use, which is biodegraded over time, comprising a blend of at least two biodegradable polymers having distinct biodegradable rates, wherein the overall degradation rate of the shaped article is different from that of the polymers comprising the blend.

8. The article of Claim 7 in the shape of a suture.

9. A method for preparing an article for temporary in vivo use, wherein biodegradation of the article comprises at least one method whereby said article is bioeroded over time, comprising:

preparing an intimate blend of at least two biodegradable polymers having distinct biodegration rates, and

forming said polymer blend into a shaped article.

10. The process of Claim 9, wherein each of said polymers is present in a weight ratio of 10%-90%.

11. The process of Claim 9, wherein said polymers are derived from monomers selected from the group consisting of ε-caprolactone, DL- or L-lactic acid, and glycolic acid and represented by one or more of the following chemical structures:

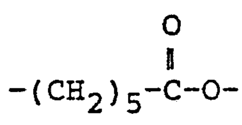

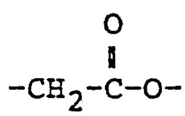

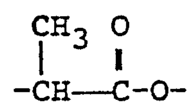

12. The process of Claim 9, wherein the article comprises a system for controlled drug delivery, wherein said polymers are blended so as to achieve a predetermined rate of biodegradation for the article.

13. The process of Claim 9, wherein said blend is shaped into the form of a microcapsule or microsphere, implant of at least 1/2 millimeter defining a sealed internal reservoir or polymer matrix in which a drug may be absorbed.

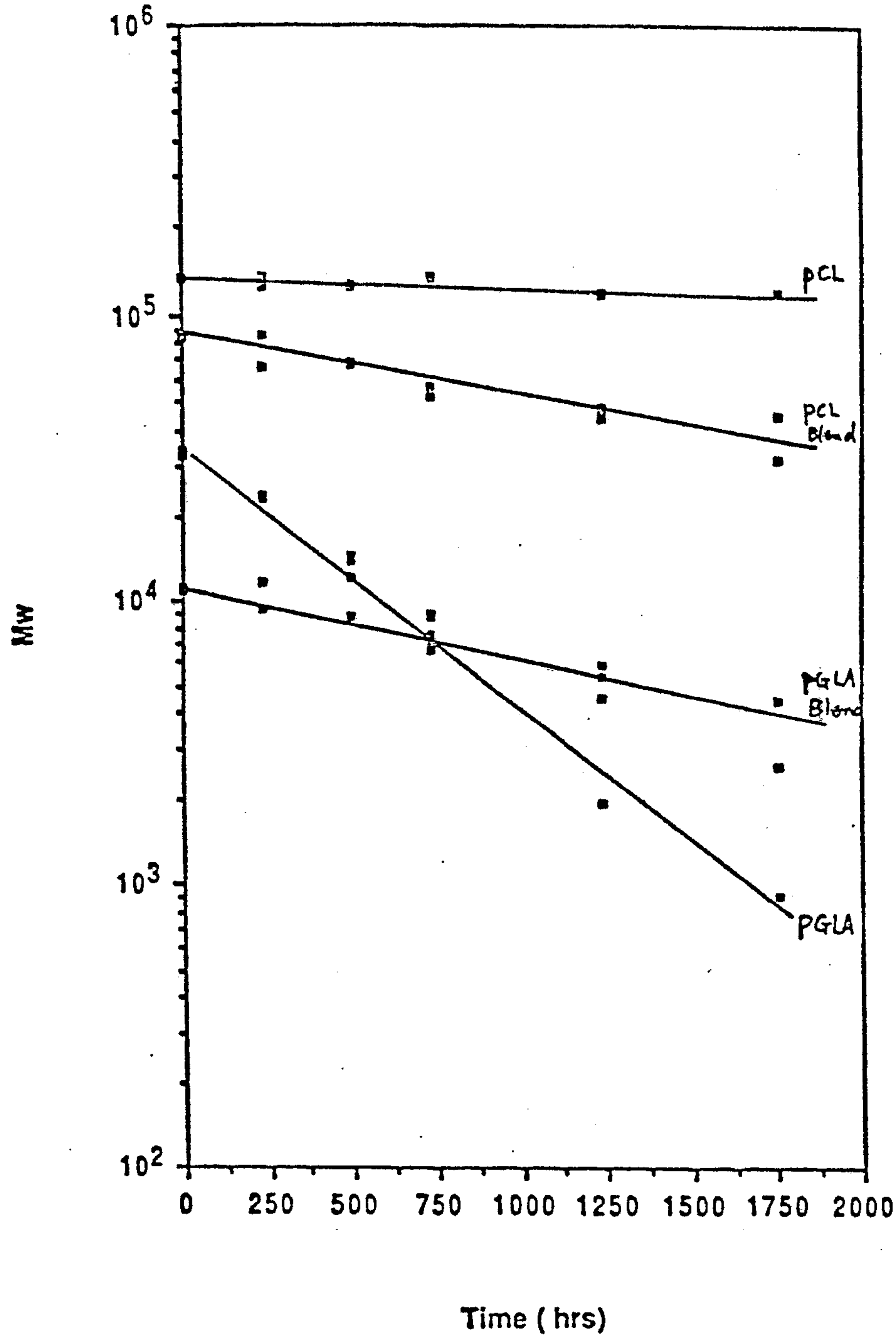


Figure 1. Changes in molecular weight ($M_w$) of poly(ε-caprolactone) and poly(glycolic-co-L-lactic acid) as pure polymers and as a 1:1 blend, in a buffered saline, pH 7.4.

European Patent Office

# EUROPEAN SEARCH REPORT

Application number

EP 88400505.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,X | EP - A1 - 0 058 481 (IMPERIAL CHEMICAL INDUSTRIES PLC)<br>* Claims 1,2,5; page 2, lines 21-27; page 9, line 20 - page 11, line 20; especially page 11, lines 12-20; page 16, line 25 - page 17, line 23; page 18, lines 20-30; example 19, lines 16-21,24; page 22, line 26 - page 23, line 9 *<br>-- | 1-13 | A 61 K 9/00<br>A 61 K 9/52<br>A 61 K 9/70 |
| X | US - A - 4 130 639 (S.W.SHALABY, P. D. JAMIOLKOWSKI)<br>* Claims 1,13,14; column 3, lines 24-44; column 11, lines 11-15 *<br>-- | 1-4,6, 7,9,10, 12,13 | |
| X | DE - A - 2 424 169 (ARTHUR D. LITTLE INC.)<br>* Claims 1,3,8,18,21; page 8, lines 4-28; page 14, last passage *<br>-- | 1-4,6, 9,13 | TECHNICAL FIELDS SEARCHED (Int. Cl.4)<br>A 61 K 9/00 |
| X | GB - A - 2 091 554 (MITSUITOATSU CHEMICALS INC.)<br>* Claims 1,2; example 5 *<br>-- | 1,2,4-7,9-13 | |
| X | DE - B2 - 2 051 580 (E.I. DU PONT DE NEMOURS AND CO.)<br>* Claim 1, column 3, lines 25-57; column 12, lines 60-68; column 14, lines 15-23 *<br>-- | 1-7,9-13 | |

The present search report has been drawn up for all claims

| Place of search | Date of completion of the search | Examiner |
|---|---|---|
| VIENNA | 01-06-1988 | MAZZUCCO |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number



EP 88400505.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 3 867 520 (S.MORI et al.)<br>* Claim 1; column 1, lines 3-60 *<br>-- | 1,2,4, 6-10, 13 | |
| X | EP - A2 - 0 138 216 (SUMITOMO CHEMICAL COMPANY LTD.)<br>* Abstract; claims 1-3,8; page 4, lines 12-22; page 6, lines 1-23; page 7, line 25 - page 8, line 18 *<br>---- | 1-7,9-13 | |

TECHNICAL FIELDS SEARCHED (Int. Cl.4)

The present search report has been drawn up for all claims

| Place of search | Date of completion of the search | Examiner |
|---|---|---|
| VIENNA | 01-06-1988 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document